(19) Europäisches Patentamt
European Patent Office
Office européen des brevets

(11) EP 0 703 211 B1

(12) # EUROPÄISCHE PATENTSCHRIFT

(45) Veröffentlichungstag und Bekanntmachung des Hinweises auf die Patenterteilung:
**10.11.1999 Patentblatt 1999/45**

(51) Int Cl.6: **C07C 43/16**, C07C 41/28

(21) Anmeldenummer: **95112073.2**

(22) Anmeldetag: **01.08.1995**

(54) **Verfahren zur Herstellung von ungesättigten Ethern**

Process for the preparation of unsaturated ethers

Procédé pour la préparation d'éthers insaturés

(84) Benannte Vertragsstaaten:
**CH DE FR GB IT LI**

(30) Priorität: **23.09.1994 DE 4433949**

(43) Veröffentlichungstag der Anmeldung:
**27.03.1996 Patentblatt 1996/13**

(73) Patentinhaber: **Degussa-Hüls Aktiengesellschaft**
**60287 Frankturt am Main (DE)**

(72) Erfinder: **Kaufhold, Manfred, Dr.**
**D-45770 Marl (DE)**

(56) Entgegenhaltungen:
**EP-A- 0 197 283**
**DE-A- 4 039 950**
**DE-C- 3 933 247**
**US-A- 2 667 517**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein neues Verfahren zur Herstellung von ungesättigten Ethern der Formel 1 aus Acetalen oder Ketalen der Formel 2 nach folgendem Reaktionsschema:

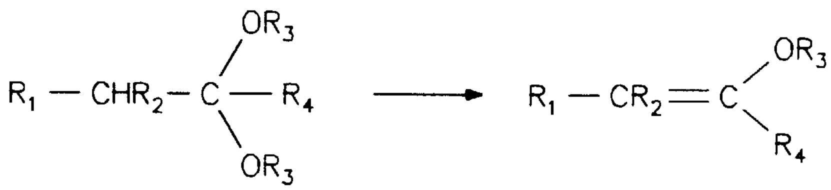

(2) (1)

Dabei kann

$R_1$ = H oder Alkyl mit 1 bis 8 C-Atomen,
$R_2$ = H, $CH_3$, $C_2H_5$ oder Cl,
$R_3$ = Alkyl mit 1 bis 8 C-Atomen und
$R_4$ = H, $CH_3$, $C_2H_5$ oder $C_3H_7$ sein.

$R_1$ und $R_4$ können aber auch zu einem 5- bis 7gliedrigen Ring miteinander verknüpft sein.
Die ungesättigten Ether sind wichtige Ausgangsverbindungen zur Herstellung von Pharmaprodukten und Riechstoffen.
Die Herstellung von ungesättigten Ethern aus Acetalen oder Ketalen ist literaturbekannt. So beschreibt US 2 667 517 ein Verfahren, das mit einem Lösemittel aus der Stoffgruppe der Kohlenwasserstoffe oder der chlorhaltigen Kohlenwasserstoffe und mit einem sauren Katalysator aus der Gruppe der aromatischen und alkylaromatischen Sulfonsäuren arbeitet. Weil bei diesem Verfahren das Lösemittel durch sich bildende hochsiedende Substanzen stark verunreinigt wird, schlägt EP-A-0 197 283 bei einem ähnlichen Verfahren ein Mineralöl als Lösemittel vor, das nach Gebrauch verbrannt wird.
Das Verfahren von DE-A-40 39 950 arbeitet ohne Lösemittel bei 160 bis 200 °C mit einem Katalysator, der aus einer Säure und einem Amin besteht. Es ist nur für bestimmte Acetale, die des Propion-, Butyr- und Valeraldehyds, gut geeignet. Auch bei diesem Verfahren wird der Katalysator mit den sich bildenden Hochsiedern verunreinigt.
Leichtsiedende Acetale, wie z. B. die Methyl- und Ethylacetale des Acetaldehyds oder des Acetons, können wegen der hohen Reaktionstemperaturen nach diesem Verfahren nicht bei Normaldruck umgesetzt werden.
Nach dem Verfahren von DE-C-39 33 241 werden kurzkettige Carbonsäuren mit Siedepunkten von weit unter 200 °C, beispielsweise Propionsäure oder Isovaleriansäure, zur Ketalspaltung eingesetzt.
Wegen der vergleichsweise niedrigen Siedepunkte eignet sich das Verfahren jedoch nur zur Spaltung von Ketalen, nicht aber zur Spaltung von Acetalen, wo Temperaturen von ca. 200 °C benötigt werden. Außerdem werden die hier verwendeten Carbonsäuren durch Veresterung mit dem entstehenden Alkohol relativ schnell verbraucht.
Aufgabe der vorliegenden Erfindung war es daher, ein einfaches Verfahren bereitzustellen, das in üblichen Rührapparaturen bei Normaldruck durchführbar ist, bei dem der Verbrauch an Katalysator gering ist, bei dem nur wenig hochsiedende Abfallprodukte entstehen und das zur Spaltung für viele verschiedene Acetale und Ketale geeignet ist.
Die Aufgabe wird erfindungsgemäß dadurch gelöst, daß man die Reaktion in einer 2,2-disubstituierten $C_7$- bis $C_{18}$-Carbonsäure bei 100 bis 250 °C durchführt und dabei den ungesättigten Ether als Destillat gewinnt.
Als Acetal der Formel 1 (für $R_4$ = H) kommen vor allem offenkettige Verbindungen, die vorzugsweise zwei gleiche Acetalreste $R_3$ aufweisen, in Betracht. Beispiele für derartige Acetale sind Dimethylacetale, Diethylacetale, Di-n-propylacetale, Di-n-butylacetale, Di-i-butylacetale, Di-n-pentylacetale, Di-i-pentylacetale, Di-n-hexylacetale und Di-i-hexylacetale von Aldehyden der allgemeinen Formel $R_1$-$CHR_2$-CHO, worin $R_1$ und $R_2$ die oben angegebene Bedeutung haben.
Beispiele derartiger Acetale sind:
Acetaldehyd-dimethylacetal, Acetaldehyd-diethylacetal, Acetaldehyd-dipropylacetal, Propionaldehyd-dimethylacetal,

Propionaldehyd-diethylacetal, Propionaldehyddipropylacetal, Propionaldehyd-dibutylacetal, Butyraldehyd-dimethylacetal, Butyraldehyd-diethylacetal, Butyraldehyd-dipropylacetal, Butyraldehyd-dibutylacetal, Butyraldehyd-dipentylacetal, Valeraldehyd-dimethylacetal, Valeraldehyd-diethylacetal, Valeraldehyd-dipropylacetal, Valeraldehyd-dibutylacetal, Valeraldehyd-dipentylacetal, Isovaleraldehyd-dimethylacetal, Isovaleraldehyd-diethylacetal, Isovaleraldehyd-dipropylacetal, Isovaleraldehyd-dibutylacetal, Isovaleraldehyd-dipentylacetal, Hexanal-dimethylacetal, Hexanal-diethylacetal, Hexanal-dipropylacetal, Hexanaldibutylacetal, Hexanal-dipentylacetal, Hexanal-dihexylacetal, 2-Ethylhexanaldimethylacetal, 2-Ethylhexanal-diethylacetal, 2-Ethylhexanal-dipropylacetal, 2-Ethylhexanal-dibutylacetal, 2-Ethylhexanal-dipentylacetal, 2-Ethylhexanal-dipentylacetal, 2-Ethylhexanal-dihexylacetal und Nonanal-dimethylacetal.

Als Beispiel für Acetale mit Chlor als Substituent seien Chloracetaldehydacetale genannt.

Als Ketale der Formel 1 (bei $R_4 \neq H$) kommen beispielsweise in Frage:
Dimethyl-, Diethyl-, Di-n-propyl-, Di-n-butyl-, Di-i-butylketale von Aceton, Butanon-2, Pentanon-2 oder -3, von Hexanon-2 oder -3, Cyclopentanon oder von Cyclohexanon. Die Ketale können auch Alkylsubstituenten oder auch Chlor als Substituenten enthalten.

Die für die Umsetzung eingesetzten Säuren haben im allgemeinen Siedepunkte von über 120 °C. Sie sollen während der Reaktion nicht mitdestillieren, obwohl sie bei den Reaktionsbedingungen unter Rückfluß sieden können. Vorzugsweise werden Carbonsäuren mit Siedepunkten von 250 bis 400 °C eingesetzt. Diese Säuren enthalten in α-Stellung zur Carboxylgruppe 2 Substituenten. Derartige Neosäuren werden beispielsweise bei der Koch'schen Synthese erhalten. Vertreter dieser Säuren sind die Neooctan-, Neononan-, Neodecan-, Neododecan- und auch die 2,2,5-Trimethyldecansäure. Aus wirtschaftlichen Gründen wählt man oft marktgängige Produktgemische, so z. B. sogenannte Neosäuren mit 9 oder mehr C-Atomen, die bei Raumtemperatur flüssig sind und sich technisch gut handhaben lassen.

Es ist ein besonderer Vorteil des erfindungsgemäßen Verfahrens, daß es ohne Katalysatoren, wie beispielsweise Schwefelsäure, p-Toluolsulfonsäure und anderen Alkylbenzolsulfonsäuren oder Phosphorsäure, auskommt. Man arbeitet vorzugsweise ohne diese Katalysatoren, obwohl man sie auch zusätzlich verwenden könnte.

Es ist sehr überraschend, daß diese Carbonsäuren katalytische Effekte besitzen, da sie schwache Säuren sind und man nach dem Stand der Technik mit starken Säuren als Katalysatoren arbeitet. Überraschenderweise erhält man fast keine hochsiedenden Nebenprodukte, die das Sumpfprodukt verunreinigen und eine Aufarbeitung erforderlich machen würden. Die hochsiedenden, verzweigten Carbonsäuren können vielmehr immer wieder in die Reaktion zurückgeführt werden, was von großem wirtschaftlichem Vorteil ist.

Das Verfahren ist außerdem sehr unempfindlich gegen Verunreinigungen. Die Acetale und Ketale können also geringe Mengen an Basen enthalten, die zur Reinigung und Abtrennung von Peroxiden verwendet werden, ohne daß dadurch die Aktivität des Mediums geringer wird.

Vorzugsweise wird das Verfahren kontinuierlich durchgeführt. Dabei ergeben sich lange Laufzeiten ohne Austausch des Mediums, obwohl beispielsweise Natronlauge mit den Einsatzprodukten in den Reaktor gelangt und einen Teil der Carbonsäure neutralisiert. Es ist ein weiterer Vorteil des Verfahrens, daß die so entstehenden Natriumsalze nicht ausfallen, sondern in der Säure gelöst bleiben. Dabei können die Salze der Carbonsäuren durch Waschen mit verdünnter wäßriger Mineralsäure auf sehr einfache Weise in die freien Carbonsäuren zurückgeführt werden.

Bei dem erfindungsgemäßen Verfahren ist normalerweise kein weiteres Lösemittel erforderlich. In besonderen Fällen, wenn es vom Einsatzprodukt her wünschenswert ist, können auch übliche, unter den genannten Bedingungen stabile Lösemittel, wie z. B. Paraffine, Alkylaromaten, oder chlorierte Kohlenwasserstoffe, wie 1-Chloralkane, zugesetzt werden.

Es ist ein großer Vorteil dieses Verfahrens, daß es keinen besonderen Reaktor benötigt, sondern in jeder Rührapparatur mit Kolonne durchgeführt werden kann.

Das erfindungsgemäße Verfahren kann schließlich auch dazu dienen, ungesättigte Ether herzustellen, in denen anschließend die Alkoxy-Gruppe mit Hilfe eines Alkohols nach dem Schema

$$CHR = CH - OR' + R''OH \rightarrow CHR = CH - OR'' + R'OH$$

ausgetauscht wird.

Zur Durchführung des erfindungsgemäßen Verfahrens legt man die hochsiedenden, verzweigten Carbonsäuren vor, erwärmt sie auf 100 bis 250 °C, vorzugsweise auf 120 bis 220 °C, und dosiert das Acetal oder Ketal kontinuierlich zu, während gleichzeitig ein Gemisch, bestehend aus ungesättigtem Ether, abgespaltenem Alkohol und nicht umgesetztem Acetal oder Ketal überdestilliert. Dieses Destillat wird - eventuell nach vorhergehender Wäsche zur Entfernung eines wasserlöslichen Alkohols - fraktioniert destilliert. Wenn Azeotrope des Ethers mit dem Alkohol auftreten, wird der Alkohol z. B. mittels Wasserwäsche entfernt.

Die Reaktion wird üblicherweise bei Normaldruck oder geringfügig erhöhtem Druck durchgeführt, es ist aber auch möglich, unter reduziertem Druck zu arbeiten.

Die nachfolgenden Beispiele sollen die Erfindung näher erläutern, ohne sie zu begrenzen.

Beispiel 1

2-Methoxypropen (MPP)

Man benutzt eine Glasapparatur, die aus einem Fünfhalskolben mit Thermometer, Rührer und Tropftrichter, dessen Ausgangsrohr bis zur tiefsten Stelle des Kolbens reicht, und mit einer aufgesetzten Destillationsapparatur besteht.

Im Fünfhalskolben werden 250 g Neononansäure und in der Destillationsvorlage 1 g Kaliumcarbonat vorgelegt. Dann werden 340 g (3,21 Mol) 2,2-Dimethoxypropan (DMP), 98,2%ig, kontinuierlich bei 130 °C in die Neononansäure gegeben.

Das Verhältnis Rücklauf zur Abnahme ist an der Destillationsapparatur auf 2 : 1 eingestellt. Nach 4 Stunden ist die Reaktion beendet, und es fallen 340 g Destillat an. Das Sumpfprodukt nimmt um 1 g zu, es ist nur schwach gelb gefärbt.

Das Destillat besitzt folgende Zusammensetzung:

| | |
|---|---|
| MPP | 48,7 % |
| Aceton | 1,2 % |
| DMP | 22,4 % |
| Methanol | 27,7 % |

Der Umsatz an DMP liegt bei 78 und die Ausbeute an MPP, auf Umsatz bezogen, bei 91 %.

Zur Aufarbeitung wird das MPP abdestilliert und zweimal mit Wasser gewaschen, um das Destillat vom Methanol zu befreien. Man erhält eine Reinheit von 99,3 %

Zur Rückgewinnung des DMP wird zum MPP-freien Destillationsrückstand n-Pentan gegeben und damit Methanol ausgekreist, wobei man zum Destillatanfall kontinuierlich Wasser zumischt, um eine gute Phasentrennung zu bekommen.

Die anfallende Wasser-Methanol-Phase wird abgetrennt und verworfen. Danach wird das Pentan abdestilliert und schließlich das DMP reindestilliert.

Beispiel 2

2-Methoxypropen (MPP)

Das im Beispiel 1 angefallene Sumpfprodukt wird in insgesamt 13 weiteren Versuchen gemäß Beispiel 1 verwendet. Die Mengenzunahme an Sumpfprodukt beträgt dabei nur 14 g. Die katalytische Wirkung des Sumpfprodukts zeigt keinen Aktivitätsverlust. Das Produkt ist flüssig und leicht beweglich. Es entstehen nur wenig Hochsieder.

Beispiel 3

Ethylvinylether

Man benutzt die im Beispiel 1 beschriebene Apparatur und arbeitet analog. Im Sumpf werden 260 g Neononansäure und in der Destillationsvorlage 3 g Natronlauge (50%ig) zur Stabilisierung vorgelegt. Dann werden 340 g Acetaldehyd-diethylacetal kontinuierlich zugegeben.

Die Reaktionstemperatur beträgt 200 °C, die Kopftemperatur 68 bis 71 °C. Der Destillatanfall, 324 g, wird bei Normaldruck destilliert. Die 1. Fraktion (159 g) enthält Ethylvinylether in einer Reinheit von 96,8 % und 2,5 % Ethanol. Die 2. Fraktion (150 g) hat folgende Zusammensetzung:

| | |
|---|---|
| Ethylvinylether | 1,6 % |
| Acetaldehyd-diethylacetal | 23,8 % |
| Ethanol | 72,3 % |

Der Umsatz bei dieser Reaktion liegt bei 89 % und die Ausbeute, bezogen auf umgesetztes Acetal, bei 86 %.

Durch Wasserwäsche erhält man den Ethylvinylether in einer Reinheit von 99,5 %.

Das Sumpfprodukt nimmt bei diesem Versuch um ungefähr 1 g zu und wird beim nächsten Versuch wieder eingesetzt.

Vergleichsbeispiel A

Man verfährt wie im Beispiel 1. Statt Neononansäure werden jedoch 300 g Dibenzoltoluol (MARLOTHERM®, Hüls AG, D-45764 Marl) und 3 g Alkylbenzolsulfonsäure (MARLON® AS 3-Säure, Hüls AG) im Kolben vorgelegt.
Bei einer Reaktionstemperatur von 200 °C werden dann 300 g Acetaldehyd-diethylacetal (98,5%ig) zudosiert.
Nach Beendigung der Reaktion beträgt die Menge an Sumpfprodukt 321 g und nach dreimaligem Einsatz 331 g. Die Zunahme liegt also nach dreimaligem Gebrauch schon bei 10 %. Der Umsatz an Acetal liegt bei 57 % und die Ausbeute, bezogen auf umgesetztes Acetal, bei 76 %.

Beispiel 4

n-Butylvinylether

Man verwendet die im Beispiel 1 beschriebene Apparatur und das hier beschriebene Verfahren mit dem Unterschied, daß man anstelle von DMP Acetaldehyd-di-n-butylacetal einsetzt und die Temperatur auf 210 °C erhöht:

250 g Neononansäure werden im Sumpf vorgelegt und
340 g Acetaldehyd-di-n-butylacetal zugegeben.

Es wird ein Umsatz von 83 % und eine Ausbeute an n-Butylvinylether, bezogen auf umgesetzten Acetal, von 70 % erreicht.

Beispiel 5

Ethyl-1-propenylether

Man verfährt wie in Beispiel 1. Anstelle von DMP setzt man jedoch Propionaldehyd-diethylacetal ein.
270 g Neononansäure werden im Sumpf vorgelegt. Bei einer Reaktionstemperatur von 200 °C werden dann 412 g Propionaldehyd-diethylacetal (97%ig) zugegeben. Ethyl-1-propenylether wird in einer Ausbeute von 97 % erhalten.

**Patentansprüche**

1. Verfahren zur Herstellung von ungesättigten Ethern der Formel 1

(1) 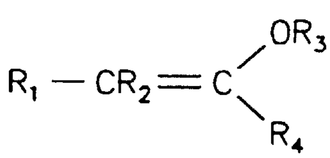

aus Acetalen oder Ketalen der Formel 2

(2)

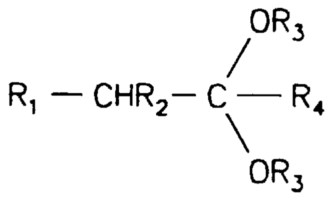

wobei

$R_1$ = H oder Alkyl mit 1 bis 8 C-Atomen,
$R_2$ = H, $CH_3$, $C_2H_5$ oder Cl,
$R_3$ = Alkyl mit 1 bis 8 C-Atomen,
$R_4$ = H, $CH_3$, $C_2H_5$ oder $C_3H_7$ sein kann und
$R_1$ und $R_4$ zu einem 5- bis 7gliedrigen Ring verbunden sein können,

dadurch gekennzeichnet,
daß man das Acetal oder Ketal der Formel 2 in einer 2,2-disbustituierten $C_7$- bis $C_{18}$-Carbonsäure auf 100 bis 250 °C erhitzt und dabei den ungesättigten Ether als Destillat gewinnt.

**2.** Verfahren nach Anspruch 1,
dadurch gekennzeichnet,
daß man auf 120 bis 220 °C erhitzt.

**3.** Verfahren nach Anspruch 1,
dadurch gekennzeichnet,
daß man keinen Katalysator zusetzt.

**4.** Verfahren nach Anspruch 1,
dadurch gekennzeichnet,
daß die Umsetzung kontinuierlich durchgeführt wird.

**Claims**

**1.** A process for preparing unsaturated ethers of the formula 1

(1)

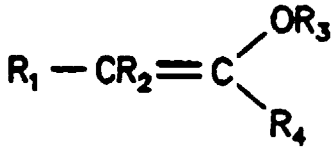

from acetals or ketals of the formula 2

(2)

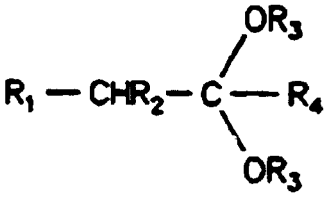

where

$R_1$ can be H or alkyl having from 1 to 8 carbon atoms,
$R_2$ can be H, $CH_3$, $C_2H_5$ or Cl,
$R_3$ can be alkyl having from 1 to 8 carbon atoms,
$R_4$ can be H, $CH_3$, $C_2H_5$ or $C_3H_7$ and
$R_1$ and $R_4$ can be joined to form a 5-membered to 7-membered ring,

characterized in that the acetal or ketal of the formula 2 is heated at from 100 to 250°C in a 2,2-disubstituted $C_7$- to $C_{18}$-carboxylic acid and the unsaturated ether is obtained as distillate.

**2.** A process according to claim 1,
characterized in that the reaction mixture is heated at from 120 to 220°C.

**3.** A process according to claim 1,
characterized in that no catalyst is added.

**4.** A process according to claim 1,
characterized in that the reaction is carried out continuously.

**Revendications**

**1.** Procédé de production d'éthers insaturés de formule 1

(1) 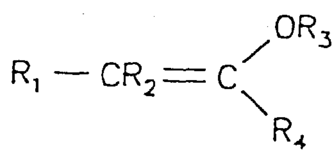

à partir d'acétals ou de cétals de formule 2

(2) 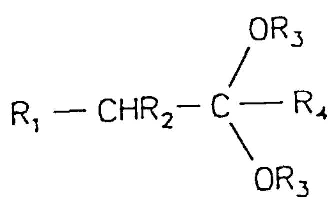

dans lesquels

$R_1$ = H ou un alkyle ayant de 1 à 8 atomes de carbone,
$R_2$ = H, $CH_3$, $C_2H_5$ ou Cl,
$R_3$ = un alkyle ayant de 1 à 8 atomes de carbone,
$R_4$ = H, $CH_3$ ou $C_2H_5$ ou $C_3H_7$, et
$R_1$ et $R_4$ peuvent également être reliés l'un à l'autre pour former un noyau à 5 à 7 chaînons,

caractérisé en ce qu'
on chauffe l'acétal ou le cétal de formule 2 dans un acide carboxylique en $C_7$ à $C_{18}$ disubstitué en 2,2 à 100 à 250°C et on obtient alors l'éther insaturé comme distillat.

**2.** Procédé selon la revendication 1,
caractérisé en ce qu'

on chauffe à 120 à 220°C.

**3.** Procédé selon la revendication 1, caractérisé en ce qu' on n'ajoute pas de catalyseur.

**4.** Procédé selon la revendication 1, caractérisé en ce qu' on conduit la réaction de façon continue.